# EUROPEAN PATENT SPECIFICATION

(11) **EP 2 968 064 B1**
(45) Date of publication and mention of the grant of the patent: **23.11.2022**
(21) Application number: 14711092.8
(22) Date of filing: 28.02.2014
(51) Int. Cl.: A61J 1/20, A61J 1/14

(54) **VIAL ACCESS CAP AND SYRINGE WITH GRAVITY-ASSISTED VALVE**
KAPPE FÜR PHIOLENZUGANG UND SPRITZE MIT SCHWERKRAFTUNTERSTÜTZTEM VENTIL
CAPUCHON D'ACCÈS DE FLACON ET SERINGUE AVEC VALVE ASSISTÉE PAR GRAVITÉ

(30) Priority: 14.03.2013 US 201313829316
(43) Date of publication of application: 20.01.2016
(60) Divisional application: 22199578.0
(73) Proprietor: CareFusion 303, Inc., San Diego, CA 92130 (US)
(72) Inventor: MANSOUR, George Michel, Pomona, California 91766 (US); PANIAN, Tyler Devin, Long Beach, California 90808 (US)
(74) Representative: Epping - Hermann - Fischer
(86) International application number: PCT/US2014/019630
(87) International publication number: WO 2014/158724

(56) References cited:
- WO-A1-2013/025946
- US-A1- 2009 254 031
- US-A1- 2010 276 034
- US-A1- 2011 062 703
- US-A1- 2011 208 128

## Description

### Cross-references to Related Applications

Not applicable.

### BACKGROUND

### Field

The present disclosure generally relates to vials and syringes, and, in particular, to vial access systems and apparatus.

### Description of the Related Art

Medications and similarly dispensed substances are typically stored in a vial that is sealed with a vial cap having an access port for injecting fluid into or removing fluid from the vial. A closure of the vial usually include a pierceable rubber stopper formed of an elastomeric material such as butyl rubber or the like. The vial cap, typically formed of metal, is crimped over the pierceable rubber stopper and a flange of the vial to hold the stopper in place in the opening of the vial. The vial cap has an opening, or access port, through which the stopper and the vial opening may be accessed. A sharp cannula, such as a needle, or the piercing end of a vial adapter is typically inserted into the access port of a vial cap to make fluid connection with the contents of a vial.

Some medications for administration, such as many types of chemotherapy preparations, are packaged and shipped in a concentrated or dehydrated form, such as, but not limited to, a concentrated liquid or a dehydrated powder. Before these dehydrated or concentrated medicaments can be administered to patients, the medicaments must be reconstituted by adding a liquid rehydration or dilution component or constituent to the concentrated or dehydrated medicament. Gases from the reconstitution process, particularly for some chemotherapy preparations, can be toxic and require a closed or non-vented arrangement during processing and administration.

WO 2013/025946 A1 describes a pressure-regulating vial adaptor of use in regulating pressure within medicinal vials.

US 2011/208128 A1 discloses drug delivery connectors for permitting and blocking fluid flow between a container and a catheter connector or other drug delivery site. Embodiments of the drug delivery connectors include a ball valve for forming a releasable seal within the drug delivery connectors.

US 2009/254031 A1 discloses a method of combining a drug in a first container and a liquid in a second container including placing the first container and the second container in fluid communication with a housing, combining the liquid and the drug in the first container, transferring the liquid and the drug from the first container to the housing, and transferring the liquid and the drug from the housing to a third container.

### SUMMARY

The invention is defined by the independent claims. Advantageous embodiments of the invention are given in the sub-claims.

In claimed embodiments, a vial access cap is disclosed that comprises a connector having an opening for providing access to a fluid pathway; a canella extending into an interior of a vial, the canella having a fluid lumen operatively coupled to the fluid pathway and a gas lumen; a gravity-assisted valve comprising a valve body defining an elongatedvalve cavity, a first port, a second port operatively coupled to the gas lumen, and a valve member movable between a sealable end of the elongated valve cavity proximal to the first port and a non-sealable end of the elongated valve cavity proximal to the second port,wherein the opening of the connector further provides sealable access to a gas pathway, the gas pathway extends from an interstitial space of the connector to the first port, wherein the first port is operatively coupled to the gas pathway, and wherein the gas pathway further extends from the second port to an interior volume of the vial.

In claimed embodiments, a system for providing fluid communications is disclosed that comprises the above described vial access cap affixed to a vial; and a syringe comprising, a connector body section defining a longitudinal axis and comprising, a tip portion for fluid ingress and egress; a second gravity-assisted valve comprising a valve body defining an elongated valve cavity generally aligned with the longitudinal axis, a first port operatively coupled to the lip portion, a second port, and a valve member movable between a sealable elongated valve cavity proximal to the first port and a non-sealable end of the elongated valve cavity proximal to the second port; a barrel section generally aligned with the longitudinal axis and slidablyengaged with a plunger, the barrel section defining a fluid icsergoir within a volume of the barrel section controllable by an end of the plunger proximal the connector body section; and a gas pathway extending from an interstitial space of the connector body section to an interior volume of the plunger defining a gas reservoir, wherein the tip portion of the syringe is removably coupled to the opening of the vial access cap, and the fluid pathway and the gas pathway of the vial access cap are operatively coupled to a fluid pathway and the gas pathway of the syringe.

It is understood that various configurations of the subject technology will become readily apparent to those skilled in the art from the disclosure, wherein various configurations of the subject technology are shown and described by way of illustration. As will be realized, the subject technology is capable of other and different configurations and its several details are capable of modification in various other respects, all without departing from the scope of the subject technology. Accordingly, the summary, drawings and detailed description are to be regarded as illustrative in nature and not as restrictive.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
**FIG. 1A** is a longitudinal cross-sectional view illustrating an example of a vial access cap, in accordance with various aspects of the present disclosure.
FIG. 1B is an enlarged, longitudinal cross-sectional view illustrating an example of a gravity- assisted valve of an vial access cap, in accordance with various aspects of the present disclosure.
FIG. 2A is a cross-sectional view illustrating an example of a syringe, in accordance with aspects of the present disclosure.
FIG. 2B is an enlarged, longitudinal cross-sectional view illustrating an example of a section of a syringe, in accordance with aspects of the present disclosure.
FIG. 2C is an enlarged, longitudinal cross-sectional view illustrating an example of a gravity-assisted valve of a syringe, in accordance with aspects of the present disclosure.
FIG. 3 is a cross-sectional view illustrating an example of a system for fluid communication having a vial access cap and a syringe in an upright orientation, in accordance with aspects of the present disclosure.
FIGS. 4A-4D are longitudinal cross-sectional views illustrating exemplary embodiments of gravity-assisted valves of a syringe and a vial access cap in an upright orientation, in accordance with aspects of the present disclosure.
FIG. 5 is a cross-sectional view illustrating an example of a system for fluid communication having a vial access cap and a syringe in an inverted orientation, in accordance with aspects of the present disclosure.
FIGS. 6A-6D are longitudinal cross-sectional views illustrating exemplary embodiments of gravity-assisted valves of a syringe and a vial access cap in an inverted orientation, in accordance with aspects of the present disclosure.

### DETAILED DESCRIPTION

Whether reconstituting a medicament or removing medication from a vial for administration, it is advantageous to provide vented and non-vented arrangements that ensure no breaches of the system contaminate the medication or expel toxic gases into the ambient environment.

Certain disclosed embodiments of vial access caps, syringes, and systems for fluid communications having vial access caps and syringes incorporating one or more exemplary gravity-assisted valves help ensure that no breaches occur and proper fluid communications are maintained during the preparation of medications in various vented and non-vented arrangements and the subsequent administration of the medications.

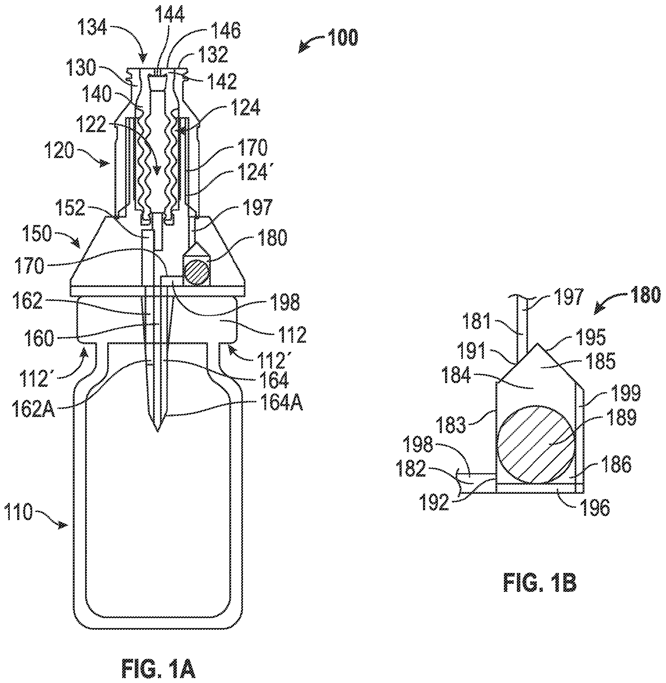

FIG. 1A illustrates an exemplary vial access cap 100 in accordance with certain embodiments. The vial access cap 1 00 is shown coupled to a vial 1 10 and comprises a connector 120, a base 1 50 including a gravity-assisted valve 180, and a canella 1 60.

Connector 120 is a needleless connector having an opening 134 adapted to a female luer fitting in accordance with certain embodiments. However, it is understood that some embodiments of the connector 120 do not have a luer tapered fitting. The connector 120 includes a body section 130 with a top surface 1 32 of the opening 1 34.

In certain embodiments, a collapsible valve 140 is disposed within the body section 130 and has a valve tip 142 that is disposed within the opening 134 such that a valve face 146 is approximately flush with the top surface 132 of the body section 130. The valve tip 1 42 comprises a slit 144 that is forced closed when the valve tip 142 is positioned in a closed configuration (as illustrated in FIG. 1A). The slit 144 will self-open when the collapsible valve 140 is compressed and the valve face 146 is displaced, by a male luer tip for example, into a wider cavity 124 of the body section 130. The slit 144 provides access to an internal pathway 122 of the collapsible valve 140. The internal pathway 122 is operatively coupled to a fluid line 152 in the base 150. In some embodiments, cavity 124 provides an interstitial space defining a gas pathway for venting or adding a gas (e.g., sterilized air) through the base 150 and into the vial 110.

Canella 160 extends into the vial 110 and is operatively coupled to the base 150. In some embodiments, canella 160 includes a fluid lumen 162 generally used for the flow of medication or diluent and a gas lumen 164 generally used for the flow of sterilized air and/or aerosolized contents discharged during the process of reconstituting a medicament. It is to be understood that the gas lumen 164 may be used in venting and non-venting arrangements. In some embodiments, an aperture 162a of the fluid lumen 162 is disposed on the canella 160 proximal to the vial rim 112 and an aperture 164a of the gas lumen 162 is disposed on the canella 160 distal to the vial rim 112.

**FIG. 1B** provides an enlarged, longitudinal cross-sectional view of the exemplary gravity-assisted valve 180 in accordance with certain embodiments. Gravity-assisted valve 180 comprises a valve body 183 defining an elongated valve cavity 184 having generally cylindrical and conical sections. Valve 180 comprises a first port 181 proximal to a sealable end 185 having a generally smooth, ramped or conical interior wall 195 of the valve cavity 184, and a second port 192 proximal to a non-sealable end 186 having a ribbed or channelized non-sealable interior wall portion 196 (e.g., a bottom of the valve body 183 having a generally circular cross-sectional area). A valve member 189 is disposed within the elongated valve cavity 184 and movable between the sealable end 185 and non-sealable end 186. In accordance with certain embodiments, the valve member is a substantially spherical element, such as, but not limited to, a ball bearing comprising a non-reactive material with respect to the type of fluid communication for which it is to be engaged covering at least an outer surface of the ball bearing.

In certain embodiments, a ribbed or channelized longitudinal interior wall portion 199 extends along at least a portion of the generally cylindrical section of the elongated valve cavity 184. The longitudinal interior wall portion 199 generally extends from the ribbed non-sealable interior wall portion 196 to proximal the generally smooth, ramped or conical interior wall 195 of the sealable end 185. The ribs or channels along the interior longitudinal interior wall portion 199 and the interior wall portion 196 proximal the non-sealable end 186 provide a gas pathway when the valve member 189 resides in the elongated valve cavity 184 proximal to the non-sealable end 186.

The first port 181 includes a first port aperture 191 disposed on the ramped or conical interior wall 195 such that the valve member 189 will seal or block flow through the aperture 191 when the valve member 189 is in a first position proximal to the sealable end 185. The first port 181 also includes a first port conduit 197 extending to a portion of the cavity 124' of the connector 120 The second port 182 includes a second port aperture 192 disposed on the generally cylindrical section of the elongated valve cavity 184 proximal the ribbed non-sealable interior wall portion 196 such that the valve member 189 cannot seal or block flow through the aperture 192 when the valve member 189 is in a second position proximal to the non-sealable end 186.

In accordance with certain embodiments described above and illustrated in FIGS. **1A** and **1B****,** vial access cap 100 and vial 110 are combined to provide an integrated vial design solution that drug compounders can use to encase medications for distribution to hospitals and pharmacies or the like. For example, a thin aluminum cover (not shown) or similar material encloses the vial access cap 100 (leaving access to the opening 134) and is crimped securely to the underside 112' of the vial rim 112. In this regard, the integrated vial design of the present disclosure eliminates the need for spikes, thereby reducing costs and increasing operational efficiencies for the drug administration entities. However, in some embodiments, the vial access cap 100 or various aspects thereof may be incorporated into a vial adapter for piercing the septum of a vial to obtain access to the medication therein.

In certain embodiments, one or more filters 170 are disposed at various locations along a gas pathway, for example, in the first port conduit 197 or in a portion of the cavity 124' of the connector 120. The filters 170 include a thin membrane or thickness of porous material, such as, but not limited to, polytetrafluoroethylene (PTFE) or other vinyl polymers having various pore sizes in some implementations.

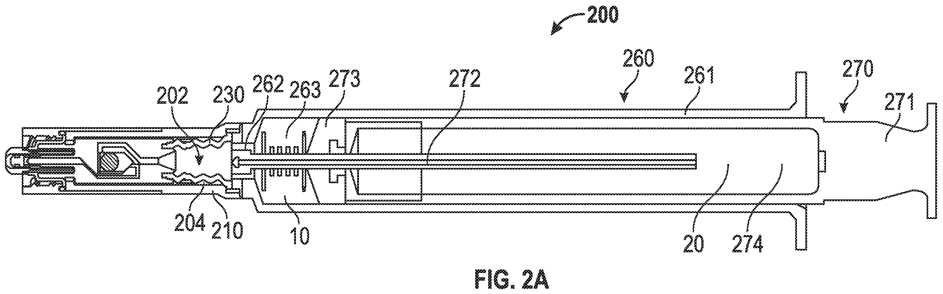

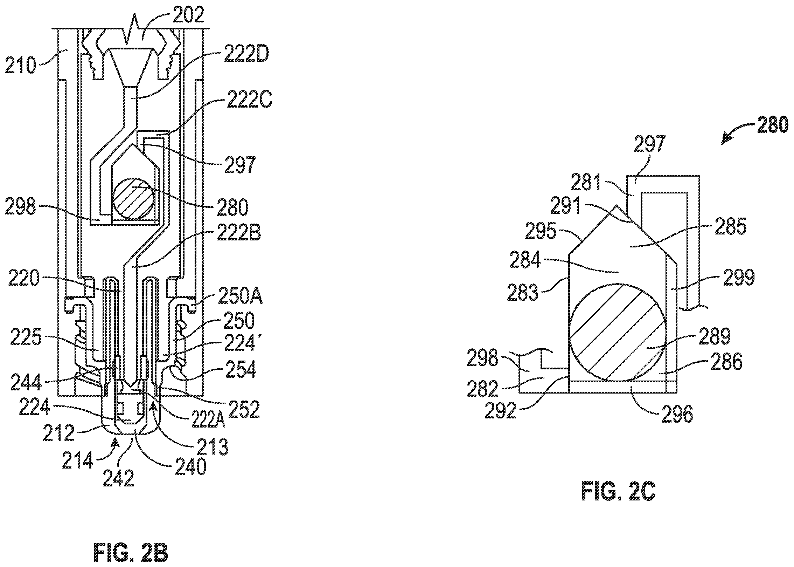

**FIGS. 2A** and **2B** illustrate an exemplary syringe 200 in accordance with certain embodiments. Syringe 200 comprises a connector body 210, a barrel 260 having a barrel tube 261, a plunger 270 having a plunger tube 271 and an interior volume 274 defining a gas reservoir, for example, a balloon (not shown) in a non-vented arrangement, or a filter (not shown) in a vented arrangement. A sealing member 273 of plunger 270, in conjunction with barrel tube 261, defines a fluid reservoir 263 and directs the flow of medication 10 (or diluent) into or out of the syringe 200. A fluid channel 262 provides a passage from the fluid reservoir 263 to the connector body 210 and a gas channel 272 provides a passage from the interior volume 274 to the connector body 210.

As shown in the enlarged, longitudinal cross-sectional view of **FIG. 2B**, the connector body 210 of the syringe 200 has a male tip or fitting 212 with a syringe port 214. In certain embodiments, the male tip (or fitting) 212 does not have a luer taper, and therefore may provide for a gas pathway to accommodate certain venting and non-venting arrangements. A valve 220 is slidably disposed within the body 210 and partially within the male tip 212. A sealing tip 240 is disposed over a tip 224 of the valve 220. The valve 220 includes accordion bellows 230 disposed within cavity 204 distal to the valve 220. The sealing tip 240 sealingly contacts the port 214 of the male tip 212 such that the external surface 242 of the sealing tip 240 is approximately flush with the external surface of the male tip 212 around the syringe port 214. The sealing tip 240 includes a second seal 244 that forms a sliding seal between the valve 220 and the male tip 212.

A fluid pathway 222 of the connector body 210 passes through the valve 220 and the sealing tip 240. In certain embodiments, the fluid pathway 222 incorporates a gravity-assisted valve 280, and comprises a longitudinal fluid pathway portion 222d that passes from the open internal cavity 202 of the bellows 230 to a lateral second port conduit 298 of the gravity-assisted valve 280, through the gravity-assisted valve 280 to a longitudinal first port conduit 297 (having a flow direction opposite that of the longitudinal fluid pathway portion 222d). The fluid pathway 222 then extends from the longitudinal first port conduit 297 of the gravity-assisted valve 280 to a lateral fluid pathway portion 222c, from the lateral fluid pathway portion 222c to a longitudinal fluid pathway portion 222b, and from the longitudinal fluid pathway portion 222b to a lateral fluid pathway portion 122a that is open to the interior of the male tip or fitting 212. The internal cavity 202 of the connector body 210 fluidly connects to the fluid channel 262 and fluid reservoir 263.

The valve 220 includes a plurality of fingers 225 that extend toward the tip 224 of the valve 220. A sliding seal 250 is disposed over a portion of the male tip 212 and the fingers 225 with a tip 252 of the sliding seal 250 in sealing contact with a recess 213 in the male fitting 212. An end 250a of the sliding seal 250 distal to the male tip 212 is captured and secured, in accordance with some embodiments, between two components that form the connector body 210. The sliding seal 250 also has a shoulder 254 disposed proximal to the tips 224' of the fingers 124. In certain embodiments, the sliding seal 250 comprises a flexible material, such as, but not limited to, and elastomeric material.

In certain embodiments, the gravity-assisted valve 280 of the syringe 200 is similar to the valve 180 shown in **FIG. 2B****,** except that the gravity-assisted valve 280 in the syringe 200 is in the fluid path and the gravity-assisted valve 180 in the vial access cap 100 is in the gas path. In some embodiments, the gravity-assisted valve 280 of the syringe 200 is placed in other portions of the fluid pathway (e.g., fluid pathway 222) and may increase the diameter or cross-sectional area for that longitudinal portion of the syringe body (e.g., connector body 210). In other embodiments, the gravity-assisted valve 280 is an adapter or connector apparatus that is connected in series with the syringe 280 as an extension of the male tip and port assembly (e.g., syringe port 214 and male tip 212).

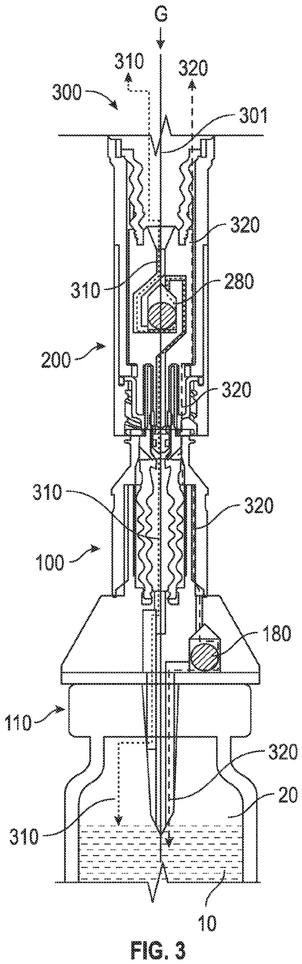

Also, it is pertinent to note the difference in conduit directions entering the first and second ports 181, 182 of the vial access gravity-assisted valve 180 and the first and second ports 281, 282 of the syringe gravity-assisted valve 280. These directional aspects with respect to the gravity-assisted valves 180, 280 have a bearing on their operation as described below with respect to **FIGS. 3****,** **4A-D****,** **5****,** and **6A-D****.**

**FIG. 3** illustrates a cross-sectional view of a system 300 for fluid communication having a vial access cap 100 and a syringe 200 in an upright orientation. In certain embodiments, the vial access cap 100 is connected to a vial 110 having fluid 10 (e.g., a medication or diluent) and gas 20 therein, and the syringe 200 is operatively coupled to the vial access cap 100. A longitudinal axis 301 is defined for the system 300 showing the alignment of the gravity-assisted valves 180, 280 with respect to gravity (G). In the upright orientation, a vial's bottom surface (not shown) is proximal to the ground and the plunger 270 of the syringe 200 is distal to the ground in a generally longitudinal arrangement.

A fluid flow path 310 extends from the vial 100 through the fluid lumen 162 to the fluid reservoir 263 of the syringe 200 (e.g., through the various pathways, conduits, and channels described and illustrated in **FIGS. 1A, 1B****,** **2A****,** **2B**, and **2C****.** A gas flow path 320 extends from the vial 100 through the gas lumen 164 to the interior volume 274 of the plunger 270 of the syringe 200 (e.g., through the various pathways, conduits, and channels described and illustrated in **FIGS. 1A, 1B****,** **2A****,** **2B,** and **2C****.**

In the upright orientation, a diluent from the fluid reservoir 263 of the syringe 200 may be added to the vial 110 to reconstitute a medicament therein. Gases from the reconstitution process, particularly for some chemotherapy preparations, can be toxic and require a closed or non-vented arrangement during processing and administration of such medication. Additionally, in some non-vented arrangements, a sterilized air may be added to the vial 110 to either increase the pressure in the vial 110 to facilitate more efficient fluid flow or bring the vial 110 to a neutral or equalized pressure without contaminating the medications therein. One or more exemplary gravity-assisted valves 180, 280 incorporated into a vial access cap 100, a syringe 200, or a system having a vial access cap 100 and a syringe 200 help ensure that no breaches occur and proper fluid communications are maintained during the preparation of medications in various non-vented arrangements and vented arrangements.

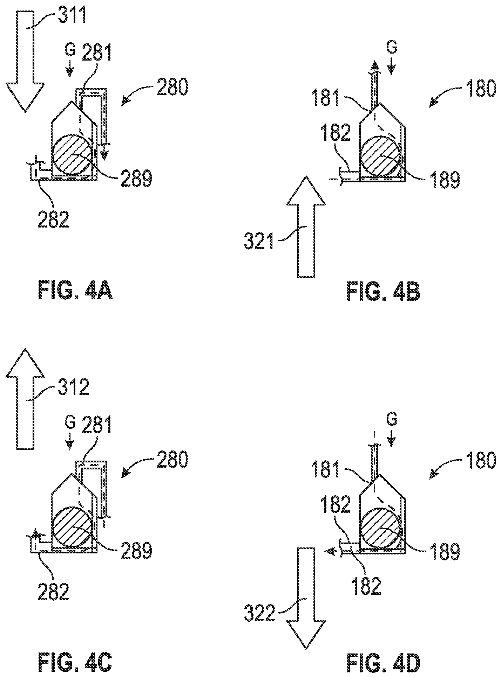

**FIGS. 4A-4D** illustrate positions of the gravity-assisted valves 180, 280 of the syringe 200 and vial access cap 100 during various operations when in the upright orientation shown in **FIG. 3****.** It is understood that, in certain embodiments, each of the valve members 189, 289 has a weight sufficient to counteract hydrostatic pressure from a gas or fluid in system 300. In general, an injection, expulsion, or suction force (or other applied or resulting force) applied to either the fluid flow path or gas flow path 320 upon the valve member 189, 289 or movement of the apparatus utilizing the gravity-assisted valve 180, 280 is required to move the valve member 189, 289 from one position to another.

In this regard, the gravity-assisted valve 180, 280 comprises at least two positions interrelated with the various operations and orientations of the system 300 in accordance with certain embodiments. For example, in a first position, the valve member 189, 289 is proximal to the sealable end 185, 285 such that the gravity-assisted valve 180, 280 is sealed at the first port 181, 281. In a second position, the valve member 189, 289 is proximal to the non-sealable end 186, 286 such that the gravity-assisted valve 180, 280 is not sealed at the first port 181, 281 and a gas or fluid can flow through the valve 180, 280.

Additionally, in the upright orientation, the gravity-assisted valves 180, 280 are biased in the second position. When biased in the second position, the gravity-assisted valves 180, 280 remain in the second position in accordance with certain aspects.

Referring to **FIG. 4A****,** when an injection force 311 is applied to the fluid flow path 310 (e.g., the plunger 270 is pushed into the barrel tube 261) while in the upright orientation, the gravity-assisted valve 280 will remain in the second position and the fluid 10 will flow from the second port 282 around the valve member 289 and out of the first port 281 in accordance with certain embodiments. Consequently, as shown in **FIG. 4B****,** when fluid 10 reaches the vial 110, an expulsion force 321 is exerted to the gas flow path 320 caused by an increase in the volume of fluid 10 (e.g., medication or diluent) entering the vial 110 or a reaction of the medicaments. As gas 20 exits the vial 110, the gravity-assisted valve 180 will remain in the second position and gas 20 will flow from the second port 182 around the valve member 189 and out of the first port 181 in accordance with certain embodiments.

In **FIG. 4C****,** when a suction force 312 is applied to the fluid flow path 310 (e.g., the plunger 270 is pulled away from the barrel tube 261) while in the upright orientation, the gravity-assisted valve 280 will remain in the second position and the fluid 10 will flow from the first port 281 around the valve member 289 and out of the second port 282 in accordance with certain embodiments. Consequently, as shown in **FIG. 4D****,** when fluid 10 is removed from the vial 110, a suction force 322 is applied to on the gas flow path 320 due to a reduction of volume of fluid 10 in the vial 110. As gas 20 enters the vial 110, the gravity-assisted valve 180 will remain in the second position and gas 20 will flow from the first port 181 around the valve member 189 and out of the second port 182 in accordance with certain embodiments. A similar operation of the gravity-assisted valve 180 as illustrated in **FIG. 4D** will result when a gas 20 is expelled from the interior volume 274 of the plunger 270 (e.g., sterilized air) though the gas flow path 320.

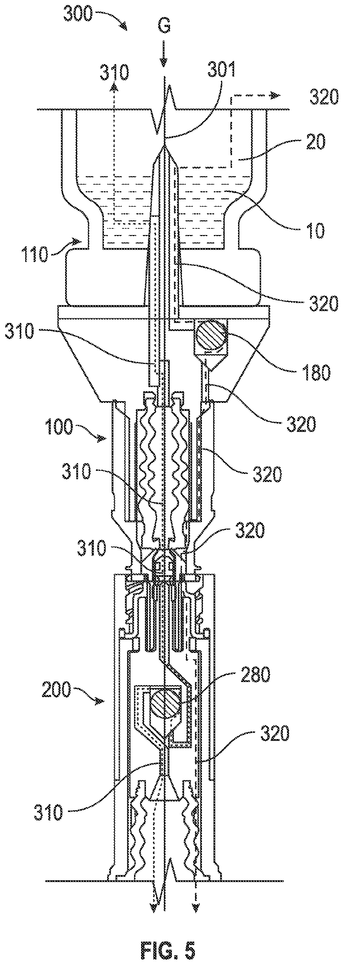

**FIG. 5** illustrates the system 300 in an inverted orientation in which the vial access cap 100 faces downwardly and is fluidly connected to the syringe 200 having its plunger 270 proximal to the ground, in accordance with certain embodiments. The inverted orientation provides an efficient manner in which medication from the vial 110 is accessed and transferred to a syringe 200, but the release of gas or fluid into the ambient environment can be hazardous and must be avoided in certain medication preparation and administration.

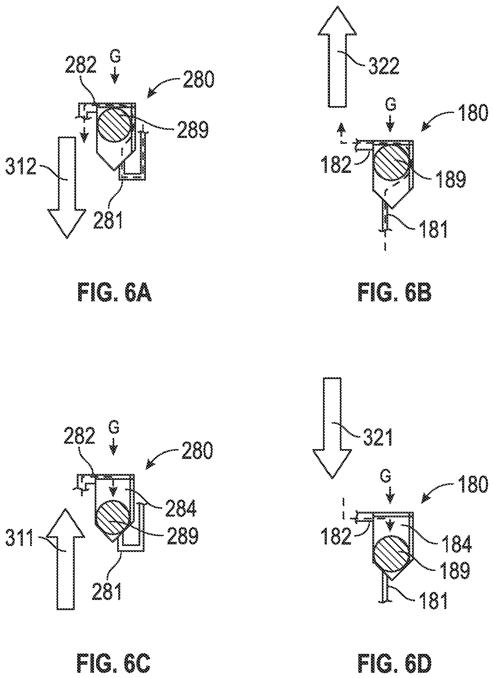

**FIGS. 6A-4D** illustrate positions of the gravity-assisted valves 180, 280 of the syringe 200 and vial access cap 100 during various operations when in the inverted orientation shown in **FIG. 5****.**

In the inverted orientation, the gravity-assisted valves 180, 280 are biased in the first position. In other words, the valve member 189, 289 is proximal to the sealable end 185, 285 such that the gravity-assisted valve 180, 280 is sealed at the first port 181, 281. When biased in the first position, the gravity-assisted valves 180, 280 may be compelled to move to the second position.

Referring now to **FIG. 6A****,** when a suction force 312 is applied to the fluid flow path 310 (e.g., the plunger 270 is pulled away from the barrel tube 261) while in the inverted orientation, the gravity-assisted valve 280 will move from the first position to the second position and the fluid 10 will flow from the first port 281 providing fluid pressure to the valve member 289 in opposition of gravity, and the fluid 10 will then flow around the valve member 289 and out of the second port 282 and into the fluid reservoir 263 in accordance with certain embodiments. In some embodiments as illustrated in **FIGS. 2C** and **6A****,** the second port aperture 292 may abut or extend into the ribbed non-sealable interior wall portion 296 so that the valve member 289 cannot fully obstruct the second port aperture 292, and the ribs or channels of the longitudinal interior wall portion 299 are arranged on a wall portion generally opposite the second port aperture 292. Hence, the resulting fluid flow extends around a longitudinally top area of the elongated valve cavity 284 and does not intersect and impede the valve member's return path to the first position into the sealable end 185 of the elongated valve cavity 284.

With reference to **FIG. 6B****,** when fluid 10 is removed from the vial 110, a suction force 322 is applied to the gas flow path 320 due to a reduction of volume of fluid 10 in the vial 110. When the suction force 322 reaches a threshold, gas 20 will be forced to enter the vial 110, thereby causing the gravity-assisted valve 180 to move from the first position to the second position. Gas 20 will flow from the first port 181 providing gas pressure to the valve member 189 in opposition of gravity, and the gas 20 will then flow around the valve member 189 and out of the second port 282 and into the vial 110 in accordance with certain embodiments.

In **FIG. 6C****,** when an injection force 311 is applied to the fluid flow path 310 (e.g., the plunger 270 is pushed into the barrel tube 261) while in the inverted orientation, the gravity-assisted valve 280 will move from the second position to the first position due to the removal of the suction force from the second port 282, the gravitational force of the valve member 289, and the injection force of the fluid 10 newly provided from the plunger 270 to the second port 282. When the gravity-assisted valve 280 is in the first position, the first port 281 will be blocked and the fluid 10 will accumulate in the elongated valve cavity and be immediately prevented from passing through fluid flow path 310 into the vial 110. Due to the proximity of the gravity-assisted valve 280 to the fluid pressure source (i.e., the injection force from the plunger 270), there is no fluid pressure build-up throughout the entire system 300 that could cause the breach of a valve, seal, conduit, or other component of the system 300 that may otherwise fail due to the pressure caused by an injection force from the plunger 270 that is not constrained.

Consequently, as shown in **FIG. 6D****,** when fluid 10 ceases to be removed from the vial 110, the suction force 322 caused upon on the gas flow path 320 likewise ceases, and the gravity-assisted valve 180 will move from the second position to the first position due to the gravitational force of valve member 189, and the expulsion force, if any, of the gas 20 or fluid 10 attempting to leave the vial 110 through the gas lumen 164. When the gravity-assisted valve 180 is in the first position, the first port 181 will be blocked and any reflux gas 20 or fluid 10 will accumulate in the elongated valve cavity 184.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

## Claims

1. A vial access cap (100) comprising:
a connector (120) having an opening (134) for providing access to a fluid pathway;
a canella (160) extending into an interior of a vial (110), the canella (160) having a fluid lumen (162) operatively coupled to the fluid pathway and a gas lumen (164); and
a gravity-assisted valve (180) comprising a valve body (183) defining an elongated valve cavity (184), a first port (181), a second port (192) operatively coupled to the gas lumen (164), and a valve member (189) movable between a sealable end (185) of the elongated valve cavity (184) proximal to the first port (181) and a non- sealable end (186) of the elongated valve cavity (184) proximal to the second port (192),
wherein the opening (134) of the connector (120) further provides sealable access to a gas pathway, the gas pathway extends from an interstitial space (124) of the connector (120) to the first port (181), wherein the first port (181) is operatively coupled to the gas pathway, and wherein the gas pathway further extends from the second port (192) to an interior volume of the vial (110).

2. The vial access cap (100) of claim 1, wherein the valve member (189) comprises a substantially spherical element.

3. The vial access cap (100) of claim 1, wherein the connector (120) defines a longitudinal axis (301), and both the valve body (183) and the canella (160) are generally aligned in parallel with the longitudinal axis (301).

4. The vial access cap (100) of claim 1, wherein the elongated valve cavity (184) includes an interior wall (195) having one or more longitudinally extending channels.

5. The vial access cap (100) of claim 4, wherein the elongated valve cavity (184) further includes an interior wall (195) disposed on the non-sealable end (186) having one or more laterally extending channels that are orthogonally aligned with respect to the one or more longitudinally extending channels.

6. The vial access cap (100) of claim 1, wherein one or more filters (170) are disposed within the gas pathway.

7. The vial access cap (100) of claim 1, wherein the interstitial space (124) of the connector (120) is included in the gas pathway.

8. The vial access cap (100) of claim 7, wherein the gravity-assisted valve (180) is configured to allow bidirectional gaseous communications between the gas lumen (164) and the interstitial space (124) of the connector (120) when the vial access cap (100) is oriented with the opening (134) of the connector (120) pointing in a generally upward direction.

9. The vial access cap (100) of claim 7, wherein the gravity-assisted valve (180) is configured to allow gaseous communications from the interstitial space (124) of the connector (120) to the gas lumen (164) when the vial access cap (100) is oriented with the opening of the connector (120) pointing in a generally downward direction.

10. The vial access cap (100) of claim 7, wherein the gravity-assisted valve (180) is configured to prohibit gaseous communications from the gas lumen (164) to the interstitial space (124) of the connector (120) when the vial access cap (100) is oriented with the opening of the connector (120) pointing in a generally downward direction.

11. A system for providing fluid communications, the system comprising:
a vial access cap of any of claims 1-10 affixed to a vial; and
a syringe (200) comprising:
a connector body section (210) defining a longitudinal axis and comprising;
a tip portion (224) for fluid ingress and egress;
a second gravity-assisted valve (280) comprising a valve body (283) defining an elongated valve cavity (284) generally aligned with the longitudinal axis, a first port (281) operatively coupled to the tip portion(224), a second port (292), and a valve member (289) movable between a sealable end (285) of the elongated valve cavity (284) proximal to the first port (281) and a non-sealable end (286) of the elongated valve cavity (284) proximal to the second port (292);
a barrel section (260) generally aligned with the longitudinal axis and slidably engaged with a plunger (270), the barrel section defining a fluid reservoir (263) within a volume of the barrel section controllable by an end of the plunger proximal the connector body section (210); and
a gas pathway extending from an interstitial space of the connector body section (210) to an interior volume (274) of the plunger (270) defining a gas reservoir,
wherein the tip portion (224) of the syringe is removably coupled to the opening of the vial access cap, and the fluid pathway and the gas pathway of the vial access cap are operatively coupled to a fluid pathway and the gas pathway of the syringe.

12. The system of claim 11, wherein the gas pathway of the vial access cap and the gas pathway of the syringe are configured for one of a venting or a non-venting arrangement.

13. The system of any of claims 11 and 12, wherein the second gravity-assisted valve is configured to allow bidirectional fluid communication between the tip portion (224) and the fluid reservoir (263) when the syringe (200) is oriented with the tip portion pointing in a generally downward direction.

14. The system of any of claims 11 and 12, wherein the second gravity-assisted valve is configured to prohibit fluid communication from the fluid reservoir (263) to the tip portion (224) when the syringe (200) is oriented with the tip portion pointing in a generally upward direction.

15. The system of any of claims 11 and 12, wherein the second gravity-assisted valve is configured to allow fluid communication from the tip portion (224) to the fluid reservoir (263) when the syringe (200) is oriented with the tip portion pointing in a generally upward direction.

## Patentansprüche

1. Kappe (100) für einen Phiolenzugang, aufweisend:
einen Verbinder (120) mit einer Öffnung (134) zum Bereitstellen eines Zugangs zu einem Fluidpfad;
eine Kanüle (160), die sich in einen Innenraum einer Phiole (110) erstreckt, wobei die Kanüle (160) ein Fluidlumen (162) hat, das mit dem Fluidpfad und einem Gaslumen (164) operativ gekoppelt ist; und
ein schwerkraftunterstütztes Ventil (180), das einen Ventilkörper (183), der einen länglichen Ventilhohlraum (184) definiert, eine erste Öffnung (181), eine zweite Öffnung (192), die mit dem Gaslumen (164) operativ gekoppelt ist, und ein Ventilelement (189) aufweist, das zwischen einem dicht verschließbaren Ende (185) des länglichen Ventilhohlraums (184) proximal zur ersten Öffnung (181) und einem nicht dicht verschließbaren Ende (186) des länglichen Ventilhohlraums (184) proximal zur zweiten Öffnung (192) bewegbar ist,
wobei die Öffnung (134) des Verbinders (120) darüber hinaus einen dicht verschließbaren Zugang zu einem Gaspfad bereitstellt, wobei sich der Gaspfad von einem Zwischenraum (124) des Verbinders (120) zur ersten Öffnung (181) erstreckt, wobei die erste Öffnung (181) mit dem Gaspfad operativ gekoppelt ist, und wobei sich der Gaspfad darüber hinaus von der zweiten Öffnung (192) zu einem Innenvolumen der Phiole (110) erstreckt.

2. Kappe (100) für einen Phiolenzugang nach Anspruch 1, wobei das Ventilelement (189) ein im Wesentlichen kugelförmiges Element aufweist.

3. Kappe (100) für einen Phiolenzugang nach Anspruch 1, wobei der Verbinder (120) eine Längsachse (301) definiert und sowohl der Ventilkörper (183) als auch die Kanüle (160) allgemein parallel zur Längsachse (301) ausgerichtet sind.

4. Kappe (100) für einen Phiolenzugang nach Anspruch 1, wobei der längliche Ventilhohlraum (184) eine Innenwand (195) mit einem oder mehreren längsverlaufenden Kanälen umfasst.

5. Kappe (100) für einen Phiolenzugang nach Anspruch 4, wobei der längliche Ventilhohlraum (184) darüber hinaus eine Innenwand (195) aufweist, die an dem nicht dicht verschließbaren Ende (186) mit einem oder mehreren seitlich verlaufenden Kanälen vorgesehen ist, die in Bezug auf den einen oder die mehreren längsverlaufenden Kanäle senkrecht ausgerichtet sind.

6. Kappe (100) für einen Phiolenzugang nach Anspruch 1, wobei ein oder mehrere Filter (170) im Inneren des Gaspfads vorgesehen sind.

7. Kappe (100) für einen Phiolenzugang nach Anspruch 1, wobei der Zwischenraum (124) des Verbinders (120) im Gaspfad enthalten ist.

8. Kappe (100) für einen Phiolenzugang nach Anspruch 7, wobei das schwerkraftunterstützte Ventil (180) so gestaltet ist, dass es bidirektionale Gasverbindungen zwischen dem Gaslumen (164) und dem Zwischenraum (124) des Verbinders (120) ermöglicht, wenn die Kappe (100) für einen Phiolenzugang mit der Öffnung (134) des Verbinders (120) so orientiert ist, dass sie in eine allgemein nach oben weisende Richtung zeigt.

9. Kappe (100) für einen Phiolenzugang nach Anspruch 7, wobei das schwerkraftunterstützte Ventil (180) so gestaltet ist, dass es Gasverbindungen vom Zwischenraum (124) des Verbinders (120) zum Gaslumen (164) ermöglicht, wenn die Kappe (100) für einen Phiolenzugang mit der Öffnung des Verbinders (120) so orientiert ist, dass sie in eine allgemein nach unten weisende Richtung zeigt.

10. Kappe (100) für einen Phiolenzugang nach Anspruch 7, wobei das schwerkraftunterstützte Ventil (180) so gestaltet ist, dass es Gasverbindungen vom Gaslumen (164) zum Zwischenraum (124) des Verbinders (120) verhindert, wenn die Kappe (100) für einen Phiolenzugang mit der Öffnung des Verbinders (120) so orientiert ist, dass sie in eine allgemein nach unten weisende Richtung zeigt.

11. System zum Bereitstellen von Fluidverbindungen, wobei das System aufweist:
eine Kappe für einen Phiolenzugang nach einem der Ansprüche 1 bis 10, die an einer Phiole befestigt ist; und
eine Spritze (200), aufweisend:
einen Verbinderkörperabschnitt (210), der eine Längsachse definiert und aufweist;
einen Spitzenabschnitt (224) für einen Fluideintritt und -austritt;
ein zweites schwerkraftunterstütztes Ventil (280), das einen Ventilkörper (283), der einen länglichen Ventilhohlraum (284) definiert, der allgemein mit der Längsachse ausgerichtet ist, eine erste Öffnung (281) operativ gekoppelt mit dem Spitzenabschnitt (224), eine zweite Öffnung (292) und ein Ventilelement (289) aufweist, das zwischen einem dicht verschließbaren Ende (285) des länglichen Ventilhohlraums (284) proximal zur ersten Öffnung (281) und einem nicht dicht verschließbaren Ende (286) des länglichen Ventilhohlraums (284) proximal zur zweiten Öffnung (292) bewegbar ist;
einen Zylinderabschnitt (260), der allgemein mit der Längsachse ausgerichtet und gleitend mit einem Kolben (270) in Eingriff steht, wobei der Zylinderabschnitt ein Fluidreservoir (263) im Inneren eines Volumens des Zylinderabschnitts definiert, das durch ein Ende des Kolbens proximal zum Verbinderkörperabschnitt (210) steuerbar ist; und
einen Gaspfad, der sich von einem Zwischenraum des Verbinderkörperabschnitts (210) zu einem Innenvolumen (274) des Kolbens (270) erstreckt, das ein Gasreservoir definiert,
wobei der Spitzenabschnitt (224) der Spritze abnehmbar mit der Öffnung der Kappe für einen Phiolenzugang gekoppelt ist, und der Fluidpfad und der Gaspfad der Kappe für einen Phiolenzugang mit einem Fluidpfad und dem Gaspfad der Spritze operativ gekoppelt sind.

12. System nach Anspruch 11, wobei der Gaspfad der Kappe für einen Phiolenzugang und der Gaspfad der Spritze für eine belüftende oder eine nicht belüftende Anordnung gestaltet sind.

13. System nach einem der Ansprüche 11 und 12, wobei das zweite schwerkraftunterstützte Ventil so gestaltet ist, dass es eine bidirektionale Gasverbindung zwischen dem Spitzenabschnitt (224) und dem Fluidreservoir (263) ermöglicht, wenn die Spritze (200) so orientiert ist, dass der Spitzenabschnitt in eine allgemein nach unten weisende Richtung zeigt.

14. System nach einem der Ansprüche 11 und 12, wobei das zweite schwerkraftunterstützte Ventil so gestaltet ist, dass es eine Fluidverbindung vom Fluidreservoir (263) zum Spitzenabschnitt (224) verhindert, wenn die Spritze (200) so orientiert ist, dass der Spitzenabschnitt in eine allgemein nach oben weisende Richtung zeigt.

15. System nach einem der Ansprüche 11 und 12, wobei das zweite schwerkraftunterstützte Ventil so gestaltet ist, dass es eine Fluidverbindung vom Spitzenabschnitt (224) zum Fluidreservoir (263) ermöglicht, wenn die Spritze (200) so orientiert ist, dass der Spitzenabschnitt in eine allgemein nach oben weisende Richtung zeigt.

## Revendications

1. Capuchon d'accès de flacon (100) comprenant :
un connecteur (120) présentant une ouverture (134) destinée à fournir un accès à un conduit de fluide ;
une canule (160) s'étendant jusque dans un intérieur d'un flacon (110), la canule (160) présentant une lumière de fluide (162) couplée opérationnellement au conduit de fluide et une lumière de gaz (164) ; et
une valve assistée par gravité (180) comprenant un corps de valve (183) définissant une cavité de valve allongée (184), un premier orifice (181), un deuxième orifice (192) couplé opérationnellement à la lumière de gaz (164), et un élément de valve (189) mobile entre une extrémité étanchéifiable (185) de la cavité de valve allongée (184) proximale par rapport au premier orifice (181) et une extrémité non étanchéifiable (186) de la cavité de valve allongée (184) proximale par rapport au deuxième orifice (192),
sachant que l'ouverture (134) du connecteur (120) fournit en outre un accès étanchéifiable à un conduit de gaz, le conduit de gaz s'étend d'un espace interstitiel (124) du connecteur (120) au premier orifice (181), sachant que le premier orifice (181) est couplé opérationnellement au conduit de gaz, et sachant que le conduit de gaz s'étend en outre du deuxième orifice (192) à un volume intérieur du flacon (110).

2. Le capuchon d'accès de flacon (100) de la revendication 1, sachant que l'élément de valve (189) comprend un élément sensiblement sphérique.

3. Le capuchon d'accès de flacon (100) de la revendication 1, sachant que le connecteur (120) définit un axe longitudinal (301), et à la fois le corps de valve (183) et la canule (160) sont sensiblement alignés en parallèle avec l'axe longitudinal (301).

4. Le capuchon d'accès de flacon (100) de la revendication 1, sachant que la cavité de valve allongée (184) inclut une paroi intérieure (195) présentant un ou plusieurs canaux s'étendant longitudinalement.

5. Le capuchon d'accès de flacon (100) de la revendication 4, sachant que la cavité de valve allongée (184) inclut en outre une paroi intérieure (195) disposée sur l'extrémité non étanchéifiable (186), présentant un ou plusieurs canaux s'étendant latéralement qui sont alignés perpendiculairement par rapport à l'un ou aux plusieurs canaux s'étendant longitudinalement.

6. Le capuchon d'accès de flacon (100) de la revendication 1, sachant qu'un ou plusieurs filtres (170) sont disposés à l'intérieur du conduit de gaz.

7. Le capuchon d'accès de flacon (100) de la revendication 1, sachant que l'espace interstitiel (124) du connecteur (120) est inclus dans le conduit de gaz.

8. Le capuchon d'accès de flacon (100) de la revendication 7, sachant que la valve assistée par gravité (180) est configurée pour permettre des communications gazeuses bidirectionnelles entre la lumière de gaz (164) et l'espace interstitiel (124) du connecteur (120) lorsque le capuchon d'accès de flacon (100) est orienté avec l'ouverture (134) du connecteur (120) pointant dans une direction sensiblement vers le haut.

9. Le capuchon d'accès de flacon (100) de la revendication 7, sachant que la valve assistée par gravité (180) est configurée pour permettre des communications gazeuses depuis l'espace interstitiel (124) du connecteur (120) vers la lumière de gaz (164) lorsque le capuchon d'accès de flacon (100) est orienté avec l'ouverture du connecteur (120) pointant dans une direction sensiblement vers le bas.

10. Le capuchon d'accès de flacon (100) de la revendication 7, sachant que la valve assistée par gravité (180) est configurée pour empêcher des communications gazeuses depuis la lumière de gaz (164) vers l'espace interstitiel (124) du connecteur (120) lorsque le capuchon d'accès de flacon (100) est orienté avec l'ouverture du connecteur (120) pointant dans une direction sensiblement vers le bas.

11. Système de mise à disposition de communications de fluide, le système comprenant :
un capuchon d'accès de flacon de l'une quelconque des revendications 1 à 10 fixé à un flacon ; et
une seringue (200) comprenant :
une section corps de connecteur (210) définissant un axe longitudinal et comprenant ;
une partie pointe (224) pour l'entrée et la sortie de fluide ;
une deuxième valve assistée par gravité (280) comprenant un corps de valve (283) définissant une cavité de valve allongée (284) sensiblement alignée avec l'axe longitudinal, un premier orifice (281) couplé opérationnellement à la partie pointe (224), un deuxième orifice (292), et un élément de valve (289) mobile entre une extrémité étanchéifiable (285) de la cavité de valve allongée (284) proximale par rapport au premier orifice (281) et une extrémité non étanchéifiable (286) de la cavité de valve allongée (284) proximale par rapport au deuxième orifice (292) ;
une section barillet (260) sensiblement alignée avec l'axe longitudinal et mise en prise de manière coulissante avec un plongeur (270), la section barillet définissant un réservoir de fluide (263) à l'intérieur d'un volume de la section barillet commandable par une extrémité du plongeur proximale par rapport à la section corps de connecteur (210) ; et
un conduit de gaz s'étendant depuis un espace interstitiel de la section corps de connecteur (210) à un volume intérieur (274) du plongeur (270) définissant un réservoir de gaz,
sachant que la partie pointe (224) de la seringue est couplée de manière amovible à l'ouverture du capuchon d'accès de flacon, et le conduit de fluide et le conduit de gaz du capuchon d'accès de flacon sont couplés opérationnellement à un conduit de fluide et au conduit de gaz de la seringue.

12. Le système de la revendication 11, sachant que le conduit de gaz du capuchon d'accès de flacon et le conduit de gaz de la seringue sont configurés soit pour un agencement avec mise à l'évent, soit pour un agencement sans mise à l'évent.

13. Le système de l'une quelconque des revendications 11 et 12, sachant que la deuxième valve assistée par gravité est configurée pour permettre une communication de fluide bidirectionnelle entre la partie pointe (224) et le réservoir de fluide (263) lorsque la seringue (200) est orientée avec la partie pointe pointant dans une direction sensiblement vers le bas.

14. Le système de l'une quelconque des revendications 11 et 12, sachant que la deuxième valve assistée par gravité est configurée pour empêcher une communication de fluide depuis le réservoir de fluide (263) vers la partie pointe (224) lorsque la seringue (200) est orientée avec la partie pointe pointant dans une direction sensiblement vers le haut.

15. Le système de l'une quelconque des revendications 11 et 12, sachant que la deuxième valve assistée par gravité est configurée pour permettre une communication de fluide depuis la partie pointe (224) vers le réservoir de fluide (263) lorsque la seringue (200) est orientée avec la partie pointe pointant dans une direction sensiblement vers le haut.
